# EUROPÄISCHE PATENTSCHRIFT

(11) **EP 0 371 013 B1**
(45) Veröffentlichungstag und Bekanntmachung des Hinweises auf die Patenterteilung: **17.11.1994**
(21) Anmeldenummer: 87906408.7
(22) Anmeldetag: 18.09.1987
(51) Int. Cl.: C08F 292/00, B01J 20/30, C12Q 1/00, C12N 1/02, G01N 30/00, G01N 33/68, G01N 33/92, G01N 33/50

(54) **REAGENZ UND VERFAHREN ZUR BINDUNG VON POLYMEREN UND MIKROORGANISMEN IN WÄSSRIGEN LÖSUNGEN**
REAGENT AND PROCESS FOR BINDING POLYMERS TO MICRO-ORGANISMS IN AQUEOUS SOLUTIONS
REACTIF ET PROCEDE DE LIAISON DE POLYMERES A DES MICRO-ORGANISMES DANS DES SOLUTIONS AQUEUSES

(43) Veröffentlichungstag der Anmeldung: 06.06.1990
(73) Patentinhaber: THIES, Karsten, D-69488 Birkenau (DE); HEUCK, Claus-Christian, F-01170 Gex (FR)
(72) Erfinder: THIES, Karsten, D-69488 Birkenau (DE); HEUCK, Claus-Christian, F-01170 Gex (FR)
(74) Vertreter: Fuchs Mehler Weiss
(86) Internationale Anmeldenummer: EP8700536
(87) Internationale Veröffentlichungsnummer: WO8902449

(56) Entgegenhaltungen:
- WO-A-84/00375
- DE-A- 2 735 179
- DE-A- 2 735 179
- US-A- 4 324 681
- US-A- 4 324 681
- Journal of Chromatography, Band 314, 1984, Elsevier Science Publishers B.V.,(Amsterdam, NL) P Maingault et al: 'Immobilization of ligands for affinitychromatography. Coupling on a spacer arm gel with N-etoxycarbonyl-2-ethoxy-1,2-dihydroquinoline as condensation agent: study of coupling conditions by meansof a radioimmunologic method', pages 445-449
- Chemical Abstracts, vol. 89, no. 15, 9 October 1978 (Columbus, Ohio, US), E. Boschetti et al.: Immobilization of ligands for affinity chromatography. A comparative study of two condensation agents: 1-cyclohexyl-3-(2-morpholinothyl)-carbodiimidemetho-p-toluene sulfonate (CMC) and N-ethoxycarbonyl-2- ethoxy-2,2-dihydroquinoline (EEDQ)", see page 256, abstract 125437s, & Biochimie 1978, 60(4), 425-7
- Chemical Abstracts, vol. 91, no. 7, 13 August 1979 (Columbus, Ohio, US), C.E. Glatz et al.: "The kinetics of binding of serum lipoproteins by immobilized heparin", see page 242, abstract 51510c & Biochim. Biophys. Acta, 1979

## Beschreibung

Die Erfindung betrifft ein Verfahren zur Herstellung eines Reagens auf Basis einer organischen oder Silica-Festphase zum Zwecke der Isolation,Präparation oder Elimination eines Polymers oder eines Mikroorganismus aus einer wässrigen Lösung.

Die Erfindung betrifft ferner ein Verfahren und Reagenz zur Isolation von Biopolymeren oder Mikrorganismen aus wässrigen Flüssigkeiten für analytische,präparative oder kurative Zwecke gegebenfalls in extrakorporalen Perfusionssystemen .

### Stand der Forschung

Isolationsverfahren zur Gewinnung ,Reinigung ,Analyse und Beseitigung von Biopolymeren oder Mikroorganismen werden heute für biotechnologische Belange, in der biochemischen und medizinischen Forschung sowie zum Zwecke der Behandlung von Arzneimitteltherapieresistenten Krankheiten eingesetzt.So gelingt es z.B.mit Hilfe von extrakorporalen Immunperfusionsverfahren pathogene Biopolymere aus menschlichem Plasma zu entfernen.Gegenüber der Plasmapheresetechnik bietet dieses therapeutische Prinzip den Vorteil der selektiven Elimination eines Pathogens ohne den gleichzeitigen Verlust anderer für einen lebenden Körper essentieller Biopolymere.

Um Isolationsverfahren ex vivo zu medizinisch- therapeutischen Zwecken nutzen zu können, müssen folgende Voraussetzungen erfüllt sein:
1.Die Elimination eines Pathogens sollte möglichst selektiv erfolgen.
2.Die Bindungskapazität der einen Stoff eliminierenden Festphase sollte optimalen praktischen Anforderungen genügen.
3.Die bindende Festphase darf keine toxischen Wirkungen zeigen.
4.Das Eliminationsverfahren darf keine physiologischen Schutzmechanismen aktivieren,deren in Folge der Behandlung eingeleitete Reaktion gegebenenfalls unerwünschte Wirkungen auslösen.

Diese Voraussetzungen stellen hohe Anforderungen an die Qualität des Materials,das mit einer z.B.dem Körper wieder zurückzuführenden Körperflüssigkeit zum Zwecke der Elimination eines Pathogens durchmischt oder durchströmt wird.

Eine spezifische Elimination kann durch eine immunologische Reaktion ,d.h. durch eine hochaffine Bindung des zu eliminierenden Biopolymers als Antigen an einen für das Antigen spezifischen Antikörper ,gegebenenfalls unter Beteiligung eines Haptens, erfolgen.Einige in biologischen Flüssigkeiten vorhandenen Biopolymere binden andererseits spezifisch an Polymere, die nicht den immunologischen System zugeordnet sind.Diese Polymere können biologischer oder synthetischer Natur sein.

Beispielsweise binden bestimmte Proteine spezifisch an Lectine (US-PS 955 279 ) oder an Polyanionen.In Gegenwart von mehrwertigen Kationen binden Biopolymere ,z.B. Lipoproteine oder gamma-Globuline, bei pH-Werten oberhalb ihres isoelektrischen Punktes an sulfatierte Polysaccharide oder Polyphosphate.

Am isoelektrischen Punkt der Lipoproteine gelingt auch eine Ausfällung aus Plasma oder Serum durch diese Polyanionen ohne Zusatz von mehrwertigen Kationen (US-PS 4 125 993).Sulfatierte Polysaccharide wurden daher auch als spezifische Reagentien zur Isolation von bestimmten Biopolymeren aus Körperflüssigkeiten verwendet.

Die Techniken zur Trennung und selektiven Elimination einzelner Komponenten wässriger Biopolymermischungen sind vielfältig (z.B.Präzipitation, Agglutination,Adsorption etc.).Für extrakorporale Eliminationssysteme durch Adsorption an eine Festphase sind verschiedene Materialien (z.B.Agarose,organische Polymerisate oder Copolymerisate,mit Polymer beschichtete Kieselgele geprüft worden (GB-PS 7936573;Stoffel,W. & & Demant,Th.,Proc.Natl.Acad.Sci.USA 78,611-615,1981;US-PS 4 10̸3 865 ).

Zur affinitätschromatographischen Trennung ist ein Verfahren bekannt, bei welchem Ethanol-lösliches N-Ethoxycarbonyl-2-ethoxy-1,2-dihydrochinolin (EEDQ) als Kondensationsreagenz verwendet wird, um Liganden auf einem Gel zu immobilisieren.

Als Ligand wird u.a. D-Tryptophanmethylester verwendet, der mittels EEDQ auf einem Gel (AC-Ultrogel) immobilisiert wird und zur Reinigung von Chymotrypsin dient (Boschetti, E,; Corgier, M.; Garelle, R., Biochimie 1978, 60(4), 425-427).

Auch Testosteron-Hemisuccinat wird als Ligand verwendet, um mittels EEDQ auf einem Gel (AH Sepharose 4B) immobilisiert zu werden (P. Maingault et al., Journal of Chromatography, 314 (1984) 445-449).

Zur chromatographischen Trennung von racemischen Gemischen wird in der US-PS-4,324,681 die Verwendung von silylierten Silicium- oder Aluminiumoxiden, die kovalent an Carboxylgruppen von chiralen Aminosäuren gebunden sind, als feste stationäre Phase beschrieben.

Als chirale Aminosäuren zur Trennung von Racematen können laut dieser Patentschrift verschiedene Klassen von Derivaten verwendet werden, einschließlich N-Alkyl und N-Acyl-Derivate, sowie Ester deren Aminosäure eine Dicarbonsäure ist oder eine Hydroxy-Gruppe enthält.

Für Trenn- und Extraktionszwecke in vitro sind Festphasen auf der Basis von Silica zwar geeignet,da die mechanischen Eigenschaften von Silica im Gegensatz zu denen organischer Festphasen bei Perfusionsverfahren zum Zwecke einer Adsorption eine hohe Durchflußrate der aufzutrennenden Polymerlösungen gewährleisten (D-PS 3 225 60̸3.5-35);wegen der bekannten Aktivierung des Gerinnungssystems,des Komplementsystems,sowie von Blutplättchen durch Silanolgruppen ist diese Festphase jedoch für eine Anwendung in einem extrakorporalen Perfusionssystem nicht verwendbar.

Es hat nicht an Versuchen gefehlt,durch kovalente Bindung eines antithrombogenen Agenz,insbesondere Heparin,an Silica die Thrombogenität der Festphase zu beseitigen.Die bisherigen Erfolge waren jedoch insbesondere für die Belange der Adsorption eines Analyts an eine Festphase in einem extrakorporalen Perfusionssystem zu medizinisch-therapeutischen Zwecken unzureichend.Zwar sind verschiedene Verfahren der kovalenten Bindung von Heparin an organische Festphasen beschrieben worden,bei denen spezifische Bindungseigenschaften des Mucopolysaccharids erhalten bleiben (Miura,Y. et al. Biomed.

Materials Res.14,619-630̸,1980̸;Ellsworth,J.L. et al.J.Lipid.Res.23, 653-659,1982;Ebert,C.D.& Kim,S.W.,Thromb.Res.26,43-57,1982);diese Verfahren erwiesen sich jedoch für eine kovalente Bindung von Heparin an Silica zur Herstellung eines Adsorbens für ex vivo Perfusionszwecke nicht anwendbar.Entweder gelang es nicht,die Thrombogenität der Silicaphase zu beseitigen, oder die Beladung der Silica-Festphase z.B. durch Kopplung von Heparin mittels Bromcyan,Cyanurchlorid, Woodward's Reagenz K,Carbodiimiden oder von aldehydderivatisiertem Heparin und damit auch die spezifische Bindungskapazität dieser Heparin-Silicaphasen gegenüber Biopolymeren,die hochaffin an Heparin binden,war zu gering.Bei einigen Kopplungsverfahren wurden spezifische Bindungseigenschaften von Heparin aufgehoben.

So wurde beispielsweise ein Immunadsorbens mit Controlled Pore Glas für Immunperfusionszwecke,in dem Heparin mit Antigenen oder Antikörpern mittels Carbodiimid an ein aminopropylsilanisiertes Silica zum Zwecke der Biokompatibilität jedoch nicht zum Zwecke der Adsorption von Biopolymeren kovalent gebunden ist ,entwickelt ( D-PS 25 32 883.0̸-35).

Typischer weise bindet diese Heparin-Silica-Phase zwar Antithrombin III, die spezifische Bindungsfähigkeit gegenüber Thrombin oder Lipoproteinen aus Plasma oder Serum ist jedoch aufgehoben .

### Ziel der Erfindung

Die der Erfindung zugrundeliegende Hauptaufgabe ist die Bereitstellung eines Verfahrens zur Herstellung eines Reagens auf der Basis einer organischen oder Silica-Festphase, das zur Isolierung eines Polymers oder Mikroorganismus aus dessen wässriger Lösung, wie z.B. eines Biopolymers aus einer Körperflüssigkeit, geeignet ist und insbesondere die Voraussetzungen zur Verwendung in einem extrakorporalen Perfusionssystem erfüllt und die vorteilhaften Eigenschaften einer als Trägermaterial dienenden Festphase wie Silica oder einer organischen Festphase mit den spezifischen Bindungseigenschaften von Polycarbonsäuren biologischer, halbsynthetischer oder synthetischer Herkunft, wie z.B. Carboxylgruppen-haltigen Mucopolysacchariden, insbesondere von Heparin, oder bestimmten Carboxylgruppen-haltigen Homo- oder Copolymeren verbindet, ohne daß sich die nachteiligen Eigenschaften des Trägermaterials bei der Verwendung ex vivo auswirken. Das Reagenz soll darüber hinaus auch für Adsorptionsprozesse in vitro für analytische oder präparative Belange verwendbar sein. Ziel der Erfindung war ferner die Herstellung eines Adsorbens zur Verwendung für die genannten Belange mit den vorteilhaften, jedoch ohne die für praktische Belange nachteiligen Eigenschaften von Heparin.

Das erfindungsgemäße Verfahren zur Herstellung eines Reagens ist dadurch gekennzeichnet, daß man
(1) Silica durch Umsetzung mit einer epoxygruppenhaltigen Silylverbindung oder einem mercaptogruppenhaltigen Silan sowie Oxypropyldiglycidether epoxysilaniert bzw. die organische Festphase durch Umsetzung mit einer Diglycidverbindung epoxidiert,
(2) das erhaltene Produkt mit einem amino- und/oder carboxylgruppenhaltigen Mono-, Oligo- oder Polymeren umsetzt, und
(3) das erhaltene Reaktionsprodukt mit einer Polycarbonsäure oder einem Derivat derselben, das in die freie Säure überführt werden kann, umsetzt.

Besondere Ausführungsformen des Verfahrens werden in den Ansprüchen 2 bis 7 beschrieben.

Erfindungsgemäß wurde festgestellt,daß ein nichtthrombogenes,stabiles Adsorbens aus Heparin und Silica hergestellt werden kann,in dem bei einer hohen Bindungskapazität der Festphase die spezifischen Bindungseigenschaften von Heparin gegenüber Biopolymeren,insbesondere gegenüber jenen des Gerinnungssystems und des Komplementsystems, sowie Lipoproteinen aus Blut,Plasma oder Serum mit einer spezifischen Dichte <1,0̸63 Kg/L erhalten sind.Die erfindungsgemäße Synthese der Festphase ist dadurch gekennzeichnet,daß saures Mucopolysaccharid, z.B. Heparin,an ein amino- und/oder Carboxylgruppen haltiges mono-, oligo-, oder polymeres Molekül,das wiederum an Silica kovalent gebunden ist,in einer Weise gekoppelt wird,daß seine spezifischen Eigenschaften z.B. gegenüber Thrombin,Antithrombin III oder - in Gegenwart erhöhter Konzentrationen freier mehrwertiger Metallkationen -Lipoproteinen mit einer spezifischen Dichte <1,0̸63 Kg/L erhalten bleiben.Bemerkenswert ist ferner,daß die Form,Partikelgröße und Porosität der erfindungsgemäß mit Heparin beschichteten Silicafestphase zwar die spezifische Bindungskapazität bestimmt, jedoch sich nicht auf die Aktivierung des Gerinnungsystems,des Komplementsystems oder der Thrombozyten im Blut auswirkt.

Ein weiterer Gegenstand der Erfindung ist die Herstellung und Verwendung einer Adsorptionsfestphase,die selektiv Low Density Lipoproteine aus Blut,Plasma oder Serum ohne Zusatz von mehrwertigen Kationen bindet.

Nach den bisherigen Vorstellungen erfolgt die Bindung von Lipoproteinen an Polyanionen über stark saure Residuen,d.h.über Sulfatgruppen oder über Phosphatgruppen .Die Bindung kommt in Gegenwart von Haptenen,z.B.mehrwertigen Metallkationen,deren Konzentrationen deutlich über den natürlicherweise in Körperflüssigkeiten vorhandenden Konzentrationen an freien,d.h.ungebundenen,Kationen liegen ,zustande.Überraschender Weise wurde festgestellt,daß Lipoproteine,insbesondere die Arteriosklerose fördernde Serumlipoproteine, (d.h.Low Density Lipoproteine,LDL, mit einer spez.Dichte 1,0̸0̸6-1,0̸63 Kg/L) in Gegenwart mehrwertiger Metallkationen,deren freie Konzentrationen geringer oder gleich den freien Kationenkonzentrationen in Körperflüssigkeiten sind,bei einem pH oberhalb ihres isoelektrischen Punktes an schwachsaure Polyanionen,z.B.Polycarboxylate gebunden werden.

Diese überraschende Beobachtung führte erfindungsgemäß zu der Synthese einer Festphase ,die sich für die Elimination von Serumlipoproteinen in vitro oder ex vivo aus Plasma oder Blut hervorragend eignet.

Gegenüber dem Eliminationsverfahren mit Hilfe einer Heparin-Silica Festphase zeichnet sich die Polycarbonsäure-Festphase,insbesondere die Polyacrylat-, Polymethacrylat- oder Polymaleinat-Festphase oder Festphasen mit Copolymerisaten der genannten Säuren dadurch aus ,daß die Adsorption von Serumlipoproteinen aus Blut,Plasma oder Serum bei einem pH 5,0̸ bis 9,5 ohne Erhöhung der Konzentrationen der mehrwertigen Metall-Kationen in der Körperflüssigkeit erfolgt.

Gegenüber einem Immunperfusionsverfahren mit an eine Festphase gekoppelten Antikörpern zeichnet sich das erfindungsgemäße Verfahren mit einem Heparin-Silica Adsorbens bzw.mit einem Polycarbonsäure-Adsorbens durch eine preiswerte Herstellung ,durch die hohe Stabilität und Lagerfähigkeit,durch die Möglichkeit der Sterilisation sowie durch eine fehlende Antigenität aus.

### Beispiel 1

### Kopplung von Heparin an Silica

15 g Kieselgel werden in 10̸0̸ ml einer gamma-Glyoidoxypropyltrialkyloxysilanlösung erwärmt.Alternativ können auch andere Epoxydgruppenhaltige Silylverbindungen verwendet werden.Anschließend wird das silanisierte Silica gewaschen und getrocknet.Das getrocknete Epoxypropylsilanisierte Silica wird mit einer wässrigen oder alkoholischen Lösung einer alpha-,beta-,oder gamma-Aminocarbonsäure,z.B Lysin oder Cystein,-alternativ können auch Oligopeptide oder Polymere von Vinylamin oder Äthylenimin oder deren Copolymerisate mit Acrylsäure Maleinsäure,Methacrylsäure oder deren Nitrile oder Amide verwendet werdenüber mehrere Stunden erwärmt,gewaschen und getrocknet.

Das Aminocarbonsaure-oxypropylsilanisierte Silica wird mit- einen Carbaminsäureester,z.B.N-Ethoxycarbonyl-2-Ethoxy-1,2-Dihydro-Chinolin, (Fa.SERVA,Heidelberg),aktivierten Heparin (50̸0̸ mg ) umgesetzt und anschließend gewaschen.Die kovalent gebundene Menge von Heparin beträgt in Abhängigkeit der für die Umsetzung angebotenen Silica Oberfläche 2 bis 10̸0̸ mg /cm³ (ccm) Festphase.

Alternativ kann Silica auch mit eine Mercaptogruppe enthaltenden Silan,z.B.Mercaptopropyltrialkyloxysilan ,silanisiert werden. 10̸ g des mercaptopropylsilanisierten Silica werden mit einer 10̸%igen Lösung von Oxypropyldiglycidäther in Tetrahydrofuran (10̸0̸ ml) bei Zimmertemperatur verrührt.Nach 12 Std.wird die Festphase gründlich mit Tetrahydrofuran und anschließend mit Äther gewaschen.Die weitere Synthese erfolgt wie zuvor beschrieben.

### Beispiel 2

### Selektive Extraktion von Low Density Lipoproteinen aus Humanplasma mit einer Heparin-Silica Festphase

Eine Säule mit einem Volumen von 10̸ cm³ (ccm) wird mit einem nach Beispiel 1 hergestellten Heparin-Silica (Korngröße 10̸0̸ Micrometer,Porengröße 10̸0̸0̸ Nanometer) gepackt.50̸ ml eines heparinisierten Humanplasma wird mit Ca⁺⁺Ionen zur Herstellung einer 20̸-50̸ millimolaren Lösung versetzt. Das Plasma wird durch die Säule mit einer Flußrate von 2ml/min passiert.Die Veränderungen der Konzentrationen einzelner Plasmakomponenten sind in der Tabelle aufgeführt:

| | Chol | Tg | HDL | ApoB | ApoA | AT III | Fib | Plasm | IgG | a-2-m |
|---|---|---|---|---|---|---|---|---|---|---|
| vor | 168 | 98 | 35 | 93 | 20̸4 | 11,5 | 286 | 29 | 1132 | 128 |
| nach | 63 | 30̸ | 32 | 0̸ | 20̸4 | 1,6 | 286 | 29 | 1146 | 121 |
| vor: Plasma vor Perfusion nach:Plasma nach Perfusion Chol:Serum-Cholesterin (mg/dl) Tg: Serum-Triglycerid (mg/dl) HDL: HDL-Cholesterin (mg/dl) ApoB:Serum-Apolipoprotein B(mg/dl) ApoA:Serum-Apolipoprotein A(mg/dl) AT III :Antithrombin III (U/ml) Fib : Fibrinogen (mg/dl) Plasm: Plasminogen(mg/dl) IgG: Immunglobulin G(mg/dl) a-2-m: alpha-2-Makroglobulin (mg/dl) | | | | | | | | | | |

Der Vergleich der Konzentrationen vor und nach Perfusion zeigt deutlich den Effekt der Elimination der Apolipoprotein B haltigen Lipoproteine, die bekanntlich eine spezifische Dichte <1,0̸63 Kg/L haben,und des Antithrombin III,während die Konzentrationen der anderen Parameter unverändert sind.

### Beispiel 3

### Biokompatibilitätsprüfung der Heparin-Silica Festphase

2,5 Liter Plasma eines Schafes, mit Ca⁺⁺ Ionen versetzt (Endkonzentration 50̸ millimolar) und mit Heparin stabilisert,werden in einem extrakorporalen System durch eine Säule von 20̸0̸ cm³ (ccm) Inhalt, die mit einer nach Beispiel 1 hergestellten Adsorberfestphase gepackt ist,ex vivo mit einer Flußrate von 20̸ bis 25 ml/min perfundiert,anschließend zur Reduktion des Calcium auf physiologische Konzentrationen dialysiert.

Nach Wiedervereinigung mit den zuvor durch Zentrifugation abgetrennten Blutzellen wird das rekonstituierte Blut dem Schaf wieder reinfundiert.Das Tier toleriert die Behandlung ohne Zeichen unerwünschter Nebenwirkungen.Die Veränderungen einzelner Plasma-Bestandteile sind in dar Tabelle aufgeführt. (Die Abkürzungen entsprechen denen für in Beispiel 2 aufgeführte Parameter)

| | Chol(mg/dl) | Tg(mg/dl) | AT III(U/ml) |
|---|---|---|---|
| Plasma vor Perfusion | 64 | 41 | 20̸,9 |
| Plasma nach Perfusion | 35 | 16 | 21,2 |

### Beispiel 4

### Adsorption von Biopolymeren an die Heparin-Silica Festphase unter Erhaltung spezifischer Merkmale

Auf eine mit einer nach Beispiel 1 hergestellten Festphase gefüllten Säule mit einem Volumen von 5 cm³ (ccm) wird 1 ml eines mit 5000 enzymatischen Einheiten Thrombin angereichertes Humanserum aufgegeben.

Danach wird die Säule mit 20̸ ml einer 0̸,15 molaren NaCl-Lösung gespült. Zur Prüfung der Enzymaktivität wird dem Kochsalz-Eluat eine Fibrinogenlösung (Endkonzentration 250̸ mg/dl) zugesetzt.

Es bildet sich kein Fibringerinnsel.Das Gerinnsel ist jedoch sofort in einem Eluat einer Fibrinogenlösung,die durch die mit 0̸,15 molarer NaCl Lösung gespülten Säule perfundiert wird,nachweisbar.

### Beispiel 5

### Herstellung einer Polyacrylat-Silica Adsorberfestphase

Ein nach Beispiel 1 hergestelltes Amino- und/oder Carboxylgruppen haltiges Silica (15 g) wird mit einer Lösung gemäß Beispiel 1 aktivierter Polyacrylsäure (0̸,5 g, Molekulargew.250̸0̸0̸0̸) umgesetzt und gewaschen. Alternativ können auch andere Carbonsäuren,z.B. Polymethacrylsäure, Polymaleinsäure oder Copolymerisate der genannten monomeren Säuren mit Äthylen oder Vinyl-,Allyl-,oder Acrylderivaten(z.B.Acrylnitril,Acrylamid) verwendet werden.In den Copolymerisaten sollte der Anteil der monomeren Säure nicht unter 30̸ Mol Prozent betragen.Alternativ können ferner reaktionsfähige Derivate der genannten Polymere bzw.Copolymere (Säurehalogenide,Säureanhydride oder Mischanhydride mit niedermolekularen Säuren z.B. Ameisensäure,Essigsäure oder Propionsäure) sowie Polyzuckersäuren biologischer,synthetischer oder halbsynthetischer Herkunft (z.B.Polygalacturonsäure,Polyglucuronsäure oder deren reaktionsfähige und in die freie Polysäure überführbare Derivate) umgesetzt werden.Das Molekulargewicht der Polysäuren ist für die kovalente Bindung unwesent lich.Zur Herstellung einer z.B. Lipoprotein adsorbierenden Festphase sollte die Moleküllänge der Polysäure nicht kürzer als 10̸0̸ nm sein.

Für andere Belange ,z.B. für die Adsorption von Thrombin,eignen sich auch Polysäuren von kürzerer Länge. Die Prüfung einer kovalenten Bindung der Polycarbonsäure an die Festphase erfolgt nach eingehender Spülung der Festphase mit einer hochkonzentrierten Salzlösung und ggfs.nach einer Hydrolyse von chemisch noch reaktionsfähigen Gruppen je nach Art der gekoppelten Polycarbonsäure mit bekannten chemischen ,physiko-chemischen und/oder biochemischen Nachweismethoden.Die kovalent gebundene Menge der Carbonsäure beträgt je nach Größe des Polymers und je nach Größe der für die Umsetzung zur Verfügung stehenden Silicaoberfläche 0̸,5 bis 50̸0̸ mg/cm³ (ccm) Festphase.

### Beispiel 6

### Herstellung einer Heparin-Polycarbonsäure Silica Festphase

15,5g einer nach Beispiel 5 hergestellten Festphase werden mit einer 20̸%igen Lösung von Essigsäureanhydrid in Tetrahydrofuran 10̸0̸ ml 4 Std. auf 50̸^{o}C erwärmt.Anschließend wird die Festphase mit Tetrahydrofuran und Äther gründlich gewaschen und in eine 10̸%ige Lysinlösung eingetragen.Die weitere Synthese erfolgt gemäß Beispiel 1.

Der Nachweis der Bindung von Heparin erfolgt gemäß Beispiel 4.

### Beispiel 7

### Adsorption von Low Density Lipoproteinen an Polycarbonsäure-Silika

Die Versuchsdurchführung erfolgt wie in Beispiel 2 beschrieben unter Verwendung einer nach Beispiel 5 hergestellten Festphase jedoch ohne Zusatz von Calcium zu der Lösung.Die Veränderungen der Konzentrationen einzelner Plasmakomponenten sind in der folgenden Tabelle aufgeführt. (Die Kennzeichnung der Komponenten entspricht den Angaben in der Tabelle des Beispiel 2.)

| | Chol | Tg | HDL-Chol | ApoB | AT III | Fib | Ca |
|---|---|---|---|---|---|---|---|
| Plasma vor Perfusion | 184 | 117 | 38 | 92 | 10̸,7 | 20̸0̸ | 1,96 |
| Plasma nach Perfusion | 75 | 94 | 34 | 2 | 9,4 | 180̸ | 1,88 |

Die Elimination von Low Density Lipoproteinen ist aus der nahezu vollständigen Elimination von Apolipoprotein B,das ausschließlich in Low Density Lipoproteinen (d.h. Lipoproteinen mit einer Dichte 1,0̸0̸6-1,0̸63 Kg/L gebunden ist) und der nur geringfügig verminderten Triglyceridkonzentrationen,die. bekanntlich überwiegend in den Very Low Density Lipoproteinen mit einer spez.Dichte < 1,0̸0̸6 Kg/L gebunden sind, ersichtlich.

### Beispiel 8

### Biokompatibilitätsprüfung der Polycarbonsäuresilica Festphase

Die gemäß Beispiel 5 bzw.6 hergestellte Festphase wird in einem extrakorporalen Perfusionssystem gemäß Beispiel 3 an einem Schaf jedoch nach Zusatz von Citrat,aber ohne Erhöhung der Ca⁺⁺Konzentrationen geprüft.Das Tier erträgt die Reinfusion des ohne zusätzliche Dialyse behandelten Plasma nach Rekombination mit den Blutzellen ohne typische Zeichen einer Kreislaufdysregulation,einer Thrombosierung oder Komplementaktivierung.

### Beispiel 9

### Herstellung einer organischen Festphase mit einer kovalent gebundenen Oligo- oder Polycarbonsäure zum Zwecke der selektiven Adsorption von Biopolymeren

10̸g einer organischen Festphase,z.B. eines Pfropfpolymerisats aus Polyvinylalkohol und Polypropylen ( HOSTAPULP,Hoechst-AG) werden mit 10̸ g von Oxypropyldiglycidäther in 10̸0̸ ml Dioxan gelöst unter Zusatz eines Lewis-Katalysators bei Zimmertemperatur umgesetzt. Alternativ können auch andere organische Matrices,z.B.Sepharose, Polyacrylamid etc.),sowie andere Diglycidverbindungen als Kopplungsreagentien umgesetzt werden.Anschließend wird ein Amino- und/oder Carboxylgruppen haltiges Mono-,Oligo-,oder Polymer (10̸g) gemäß Beispiel 1 an die Festphase gekoppelt.Die kovalente Verknüpfung mit einer Polycarbonsäure,z.B.Polyacrylsäure, bzw.deren reaktionsfähige Derivate oder mit Heparin erfolgt gemäß Beispiel 5 bzw.Beispiel 1.Alternativ kann an eine amino- oder mercaptoderivatisierte organische Festphase (z.B.Fraktogel,Merck GmbH) die Polycarbonsäure gemäß Beispiel 1 bzw.Beispiel 5 kovalent gebunden werden.

Die Adsorptionseigenschaften und Biokompatibilitätseigenschaften der Festphasen entsprechen den Beispiel 7 bzw.Beispiel 2 beschriebenen.

### Beispiel 10̸

### Quantitative Bestimmung von Low Density Lipoprotein Cholesterin

50̸ ml eines Humanserums werden durch eine mit einer gemäß Beispiel 9 hergestellten Adsorberfestphase gefüllten Säule mit einem Säulenvolumen von 10̸ cm³ (ccm) perfundiert. Die Säule wird anschließend mit 10 ml einer 1 molaren NaCl Lösung gewaschen.Die Lipidkonzentrationen werden im nativen Serum, Im Durchlauf und in dem Eluat zur Berechnung der adsorbierten Lipidmengen bestimmt.Zum Vergleich werden die Cholesterinmengen in den einzelnen Lipoproteinfraktion z.B. nach Friedewald ermittelt.

| | Nativserum | Durchlauf | Differenz |
|---|---|---|---|
| Serum-Triglycerid (mg) | 68,5 | 46,9 | 21,6 |
| Serum-Cholesterin (mg) | 93,4 | 27,6 | 65,8 |
| HDL-Cholesterin (mg) | 13,8 | 13,8 | 0̸ |
| LDL-Cholesterin (mg) | 65,7 | | |

Aus den Daten in der Tabelle wird ersichtlich,daß die adsorbierte Cholesterinmenge der nach Friedewald ermittelten LDL-Cholesterin menge entspricht.

### Beispiel 11

### Adsorption von Mikroorganismen an eine Polycarbonsäure Festphase

1 ml einer wässrigen Suspension von einem Escherichia coli hämolyticus Stamm (0̸,1 Million Keime/ 10̸ Mikroliter) wurde einer mit einer gemäß Beispiel 9 hergestellten Festphase gefüllten Säule mit einem Volumen von 10̸ cm³ (ccm) aufgetragen. Alternativ wurde ein Versuch mit einer Säule, die eine gemäß Beispiel 6 hergestellte Festphase enthielt,ausgeführt.

Im Eluat der Säulen konnten keine Keime nachgewiesen werden.

Die Säulen wurden bei 37^{o}C über mehrere Tage inkubiert.Die tägliche Prüfung von Stoffwechselprodukten bestätigte die Aktivität der Mikroorganismen über einen Zeitraum von mindestens 7 Tagen.

## Patentansprüche

1. Verfahren zur Herstellung eines Reagens auf Basis einer organischen oder Silica-Festphase zur Isolierung eines Polymers oder Mikroorganismus aus einer wäßrigen Lösung in vitro und/oder ex vivo, dadurch gekennzeichnet, daß man
(1) Silica durch Umsetzung mit einer epoxygruppenhaltigen Silylverbindung oder einem mercaptogruppenhaltigen Silan sowie Oxypropyldiglycidether epoxysilaniert bzw. die organische Festphase durch Umsetzung mit einer Diglycidverbindung epoxidiert,
(2) das erhaltene Produkt mit einem amino- und/oder carboxylgruppenhaltigen Mono-, Oligo- oder Polymeren umsetzt, und
(3) das erhaltene Reaktionsprodukt mit einer Polycarbonsäure oder einem Derivat derselben, das in die freie Säure überführt werden kann, umsetzt.

2. Verfahren gemäß Anspruch 1, dadurch gekennzeichnet, daß man als amino- und/oder carboxylgruppenhaltiges Monomeres eine Diaminocarbonsäure, Mercaptoaminocarbonsäure bzw. -dicarbonsäure, und als amino- und/oder carboxylgruppenhaltiges Oligomeres oder Polymeres ein Homo- oder Copolymerisat von Galacturonsäure, Glucuronsäure, Ethylenimin, Galactosamin, Glucosamin oder Gemische derselben einsetzt.

3. Verfahren gemäß einem der Ansprüche 1-2, dadurch gekennzeichnet, daß die Polycarbonsäure biologischer, halbsynthetischer oder synthetischer Herkunft und linear oder verzweigt ist.

4. Verfahren gemäß einem der Ansprüche 1-3, dadurch gekennzeichnet, daß die Länge der Polycarbonsäure 10 bis 500 000 Nanometer beträgt.

5. Verfahren gemäß einem der Ansprüche 1-4, dadurch gekennzeichnet, daß die Polycarbonsäure ein carboxylgruppenhaltiges Mucopolysaccharid und/oder ein Polymer oder Copolymer aus Acrylsäure, Methacrylsäure und/oder Maleinsäure mit einem nichtionischen Allyl-, Acryl- oder Vinylmonomerderivat ist, wobei der Monomerenanteil der Säure mindestens 30 Mol-% beträgt.

6. Verfahren gemäß einem der Ansprüche 1-5, dadurch gekennzeichnet, daß die kovalent zu bindende Polycarbonsäure durch einen Carbaminsäureester aktiviert oder in Form ihres Halogenids, ihres Anhydrids oder Mischanhydrids mit niedermolekularen Säuren oder ihres Methyl-, Ethyl- oder Propylesters umgesetzt wird.

7. Verfahren gemäß Anspruch 6, dadurch gekennzeichnet, daß der Carbaminsäureester 1-Ethoxy-N-ethoxycarbonyl-dihydrochinolin ist.

8. Reagens zum Zwecke der Isolation eines Polymers oder eines Mikroorganismus aus einer wässrigen Lösung in vitro oder ex vivo, hergestellt nach einem der Verfahren gemäß Ansprüchen 1 bis 7.

9. Verfahren zur Elimination von Biopolymeren oder Mikroorganismen aus einer wässrigen Lösung, dadurch gekennzeichnet, daß die Elimination durch Adsorption an dem Reagens gemäß Anspruch 8 als Matrix erfolgt.

10. Verfahren gemäß Anspruch 9, dadurch gekennzeichnet, daß die Elimination eines Biopolymers aus einer Körperflüssigkeit in vitro und/oder ex vivo in einem extrakorporalen Perfusionssystem erfolgt.

11. Verfahren gemäß Anspruch 10, dadurch gekennzeichnet, daß die Biopolymeren Serumlipoproteine und/oder Biopolymere, die am plasmatischen Gerinnungssystem mitwirken, sind.

12. Verfahren zur Bestimmung der Konzentration eines Analyts in einer wässrigen Lösung, dadurch gekennzeichnet, daß die Konzentration des Analyts oder eines seiner Bestandteile direkt oder aus der Differenz der Konzentration des Analyts oder eines seiner Bestandteile vor und nach der Elimination des Analyts aus der Lösung mit Hilfe eines Reagens gemäß Anspruch 8 ermittelt wird.

13. Verfahren gemäß Anspruch 12, dadurch gekennzeichnet, daß der zu bestimmende Analyt Low Density Lipoprotein aus Blut, Plasma oder Serum oder ein Bestandteil desselben ist.

14. Verfahren gemäß Anspruch 13, dadurch gekennzeichnet, daß die Konzentration des Low Density Lipoproteins in Gegenwart von mehrwertigen Metallkationen, deren freie Konzentrationen geringer als die oder gleich den freien Kationenkonzentrationen in der Körperflüssigkeit sind, bei einem pH-Wert oberhalb ihres isoelektrischen Punktes ermittelt wird.

## Claims

1. A method for preparing a reagent on the basis of an organic or silica solid phase for isolating a polymer or microorganism from an aqueous solution in vitro and/or ex vivo, characterised in that
(1) silica is epoxysilanised by reaction with a silyl compound containing epoxy groups or a silane containing mercapto groups and oxypropyl diglycide ether or the organic solid phase is epoxidised by reaction with a diglycide compound,
(2) the resulting product is reacted with a monomer, oligomer or polymer containing amino groups and/or carboxyl groups, and
(3) the resulting reaction product is reacted with a polycarboxylic acid or a derivative thereof, which can be converted into the free acid.

2. A method according to Claim 1, characterised in that a diamino carboxylic acid, mercaptoamino carboxylic acid or dicarboxylic acid is used as the monomer containing amino groups and/or carboxyl groups, and a homopolymer or copolymer of galacturonic acid, glucuronic acid, ethylene imine, galactosamine, glucosamine or mixtures thereof are used as the oligomer or polymer containing amino and/or carboxyl groups.

3. A method according to one of Claims 1 - 2, characterised in that the polycarboxylic acid is of biological, semi-synthetic or synthetic origin and is linear or branched.

4. A method according to one of Claims 1 - 3, characterised in that the length of the polycarboxylic acid is 10 to 500,000 nanometres.

5. A method according to one of Claims 1 - 4, characterised in that the polycarboxylic acid is a mucopolysaccharide containing carboxyl groups, and/or a polymer or copolymer of acrylic acid, methacrylic acid and/or maleic acid with a non-ionic allyl, acrylic or vinyl monomer derivative, the monomer content of the acid being at least 30 mole percent.

6. A method according to one of Claims 1 - 5, characterised in that the polycarboxylic acid which is to be bonded covalently is activated by a carbamic acid ester, or is reacted in the form of its halide, anhydride or mixed anhydride with low-molecular acids or its methyl, ethyl or propyl ester.

7. A method according to Claim 6, characterised in that the carbamic acid ester is 1-ethoxy-N-ethoxycarbonyl-dihydroquinoline.

8. A reagent for the purpose of isolating a polymer or a microorganism from an aqueous solution in vitro or ex vivo, prepared according to one of the methods of Claims 1 to 7.

9. A method for eliminating biopolymers or microorganisms from an aqueous solution, characterised in that the elimination takes place by adsorption on the reagent according to Claim 8 as matrix.

10. A method according to Claim 9, characterised in that the elimination of a biopolymer from a body fluid in vitro and/or ex vivo takes place in an extracorporeal perfusion system.

11. A method according to Claim 10, characterised in that the biopolymers are serum lipoproteins and/or biopolymers which cooperate in the plasmatic coagulation system.

12. A method for determining the concentration of an analyte in an aqueous solution, characterised in that the concentration of the analyte or of one of its constituents is determined directly or from the difference in the concentration of the analyte or of one of its constituents before and after the elimination of the analyte from the solution with the aid of a reagent according to Claim 8.

13. A method according to Claim 12, characterised in that the analyte to be determined is low density lipoprotein from blood, plasma or serum or is a constituent thereof.

14. A method according to Claim 13, characterised in that the concentration of the low density lipoprotein is determined in the presence of polyvalent metal cations, the free concentrations of which are less than or equal to the free cation concentrations in the body fluid, at a pH value above their isoelectric point.

## Revendications

1. Procédé de préparation d'un réactif à base d'une phase solide organique ou de silice, pour l'isolement d'un polymère ou d'un micro-organisme à partir d'une solution aqueuse, in vitro et/ou ex vivo, caractérisé en ce que :
(1) on réalise une époxysilanisation de la silice par réaction avec un composé silylé contenant un radical époxyde ou un silane contenant un radical mercapto ainsi qu'un oxypropyldiglycidyléther ou on réalise une époxydation de la phase solide organique par réaction avec un composé diglycidyle;
(2) on transforme le produit obtenu avec un mono-, oligo- ou polymère contenant des radicaux amino et/ou carboxyle, et
(3) on transforme le produit de réaction obtenu avec un poly(acide carboxylique) ou un dérivé de celui-ci, qui peut être converti en l'acide libre.

2. Procédé suivant la revendication 1, caractérisé en ce que l'on utilise comme monomère contenant des radicaux amino et/ou carboxyle, un acide ou diacide diaminocarboxylique, un acide ou diacide mercaptoaminocarboxylique, et comme oligomère ou polymère contenant des radicaux amino et/ou carboxyle, un homo- ou copolymère de l'acide galacturonique, de l'acide glucuronique, de l'éthylèneimine, de la galactosamine, de la glucosamine ou un mélange de ceux-ci.

3. Procédé suivant la revendication 1 ou 2, caractérisé en ce que le poly(acide carboxylique) est d'origine végétale, biologique, semi-synthétique ou synthétique, et linéaire ou ramifié.

4. Procédé suivant l'une quelconque des revendications 1 à 3, caractérisé en ce que la longueur du poly(acide carboxylique) atteint 10 à 500 000 nm.

5. Procédé suivant l'une quelconque des revendications 1 à 4, caractérisé en ce que le poly(acide carboxylique) est un mucopolysaccharide contenant des radicaux carboxyle et/ou un polymère ou copolymère d'acide acrylique, d'acide méthacrylique et/ou d'acide maléique avec un dérivé allylique, acrylique ou vinylique non ionique, dans lequel la quantité en monomère d'acide atteint au moins 30% en poids.

6. Procédé suivant l'une quelconque des revendications 1 à 5, caractérisé en ce que le poly(acide carboxylique) à fixer de manière covalente est activé par un ester de l'acide carbamique ou transformé sous forme de son halogénure, de son anhydride ou d'un anhydride mixte avec des acides de poids moléculaire faible ou de ses esters méthylique, éthylique ou propylique.

7. Procédé suivant la revendication 6, caractérisé en ce que l'ester de l'acide carbamique est la 1-éthoxy-N-éthoxycarbonyldihydroquinoléine.

8. Réactif pour isoler un polymère ou un micro-organisme à partir d'une solution aqueuse, in vitro ou ex vivo, préparé suivant l'un quelconque des procédés suivant les revendications 1 à 7.

9. Procédé d'élimination de biopolymères ou de micro-organismes à partir d'une solution aqueuse, caractérisé en ce que l'élimination résulte de l'adsorption sur un réactif suivant la revendication 8, comme matrice.

10. Procédé suivant la revendication 9, caractérisé en ce que l'élimination d'un biopolymère à partir d'un fluide corporel a lieu in vitro et/ou ex vivo, dans un système de perfusion extracorporel.

11. Procédé suivant la revendication 10, caractérisé en ce que les biopolymères sont des lipoprotéines sériques et/ou des biopolymères qui participent au système plasmatique de coagulation.

12. Procédé de détermination de la concentration d'un analyte dans une solution aqueuse, caractérisé en ce que la concentration de l'analyte ou d'un de ses composants est établie de manière directe ou par différence des concentrations de l'analyte ou d'un de ses composants avant et après élimination de l'analyte de la solution au moyen d'un réactif suivant la revendication 8.

13. Procédé suivant la revendication 12, caractérisé en ce que l'analyte à déterminer est une lipoprotéine à faible densité provenant du sang, du plasma ou du sérum, ou un de ses composants.

14. Procédé suivant la revendication 13, caractérisé en ce que la concentration de la lipoprotéine à faible densité est déterminée en présence de cations métalliques polyvalents dont la concentration à l'état libre est inférieure à ou égale aux concentrations en cations libres dans le fluide corporel, à un pH supérieur à son point isoélectrique.
